# EUROPEAN PATENT SPECIFICATION

(11) **EP 0 427 949 B1**
(45) Date of publication and mention of the grant of the patent: **15.06.1994**
(21) Application number: 90118947.2
(22) Date of filing: 04.10.1990
(51) Int. Cl.: A61B 17/11

(54) **Apparatus for performing anastomosis**
Vorrichtung zum Vernähen von Anastomosen
Appareil pour l'application d'anastomoses

(30) Priority: 17.10.1989 CH 3832/89
(43) Date of publication of application: 22.05.1991
(73) Proprietor: BIEFFE MEDITAL S.A., CH-6900 Lugano (CH)
(72) Inventor: Segato, Giuseppe, I-36057 Arcugnano (VI) (IT)
(74) Representative: Incollingo, Italo

(56) References cited:
- EP-A- 0 173 451
- WO-A-81/00668
- WO-A-87/06448
- FR-A- 2 443 239
- US-A- 4 485 817
- US-A- 4 667 673

## Description

The present invention refers to an apparatus for applying surginal staples in surgical operations, including : A) at one end a head part for the application of said staples, comprising at least a cartridge (7) with associated staples and hollow punches (10) and anvil; B) at the other end a control part provided with knob (21) and lever (23); and C) a flexible intermediate part transmitting controls and stresses from B) to A). As it is well-known, a stapler for surgical operations is used for carrying out the mechanical connection (followed by the natural biological connection) of two open sections of ducts of the human or animal body. The said two sections are prevesiouly prepared with the aid of an equipment suitable for forming onto its ends a binding of the type said "bag purse". The stapler is introduced into one of the two sections through the opening that is more easily accessible (e.g. in the intestine through the rectum opening) until its end reaches the operation zone where the separated sections are pressed in a cartridge fixed to the head and are joined mechanically through staples (supplied by the cartridge). The exceeding organic tissue part in the inside of the duct is removed by punching made by the same stapler after the stapling. In the following, by extracting the stapler out of the opening (e.g. from rectum) it is recovered also the exceeding tissue cut by the hollow punch. After a certain time it is got the organic welding in the connection zone of the two sections, the removal of the tissue mechanically joined through the staples and its elimination by natural way (e.g. by excrements).

Systems and equipments for carrying out this important surgical operation are widely described in literature.

E.g. US-A-4 485 817 claims a stapler according the preamble of claim 1 including: A) means for applying staples to the tissue (head) including a member for the application of the staples (cartridge) and an anvil; B) means for controlling the elements of the said head; and C) drive means between A) and B) including a flexible shaft that can be bent during the insertion and can be adapted to the configuration of the opening of the human body and can hold the assumed configuration during the operation.

The patented equipment has surely various advantages in particular that of providing a surgical stapler with flexible shaft, but it shows also many drawbacks, among which it can be noted: the irremovibility of the head, the necessity of adjusting it "in situ", its operation by compression and, above all, the necessity of having a rather great diameter of the articulated pipe to be introduced into the duct to be sutured. In all cases this diameter cannot be reduced for making easier the insertion.

Further the control is rather complex and its elastic flexibility and memory are limited.

Just for fixing the ideas, the scheme of Figure 18 shows the operative method of the conventional equipments included the one according to US-A-4 485 817 that foresees: the phase I of inserting the stapler with closed head (with diameter e.g. of 32 mm); the phase II of opening the head in the inside of the intestine duct (turning the pushbutton of the control device); the phase III of inserting the tobacco purse, in IV) the closing of the cartridge (turning the pushbutton in opposite direction) and in V) the stapling and hollow punching. The wole operation is painful for the patient and requires also notable efforts by the surgeon who must make all these phases and moves acting manually. FR-A-2443239 describes a surgical instrument at least a portion of which can be introduced into a duct for the end to end connection of the two sections of a tube, said instrument comprising a bar, a first rigid body to hold and guide the staples mounted on said bar, and a second rigid part supporting the anvil also mounted on the bar. The instrument is introduced into the duct with the head at least partially mounted on the bar. A first aim of the present invention is now to provide a stapler that does not show the above mentioned drawbacks; in particular it is not provided with the head in the insertion phase and therefore shows a little diameter and the possibility of preadjusting the head at the ouside.

A second aim is to enable a method for the execution of the sutures in short times, with the maximum operative safety, without dangers and occasions of pollutions, and without the necessity of causing the intervention of the surgeon in all phases. These aims and other ones are achieved by the apparatus having the characteristics specified in the claims.

The different characteristics and advantages of the invention shall appear better in the description of the (preferred and unlimiting) embodiment form shown in the annexed drawings, in which:

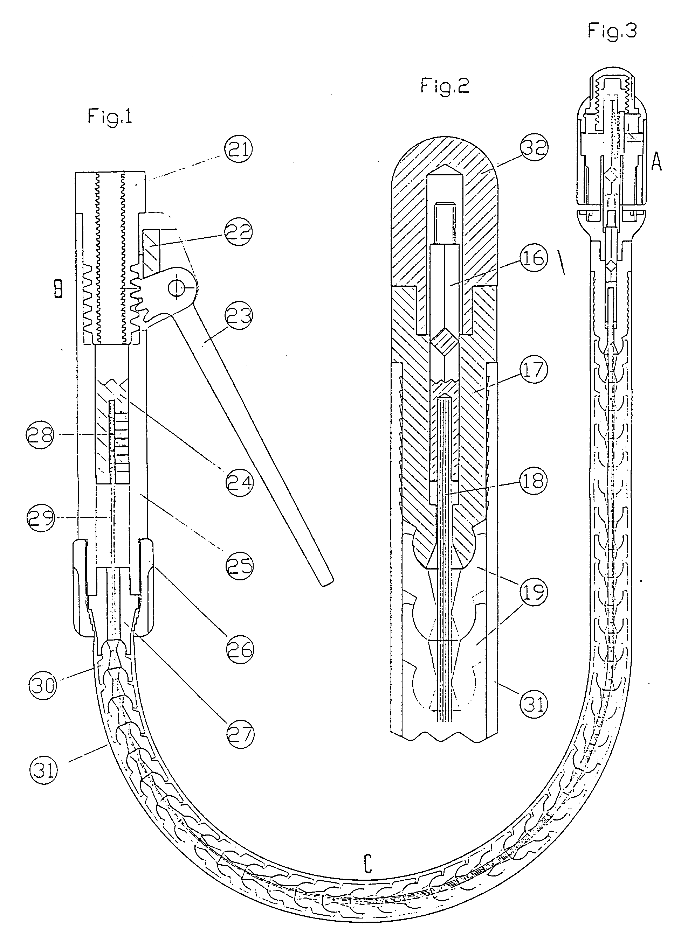

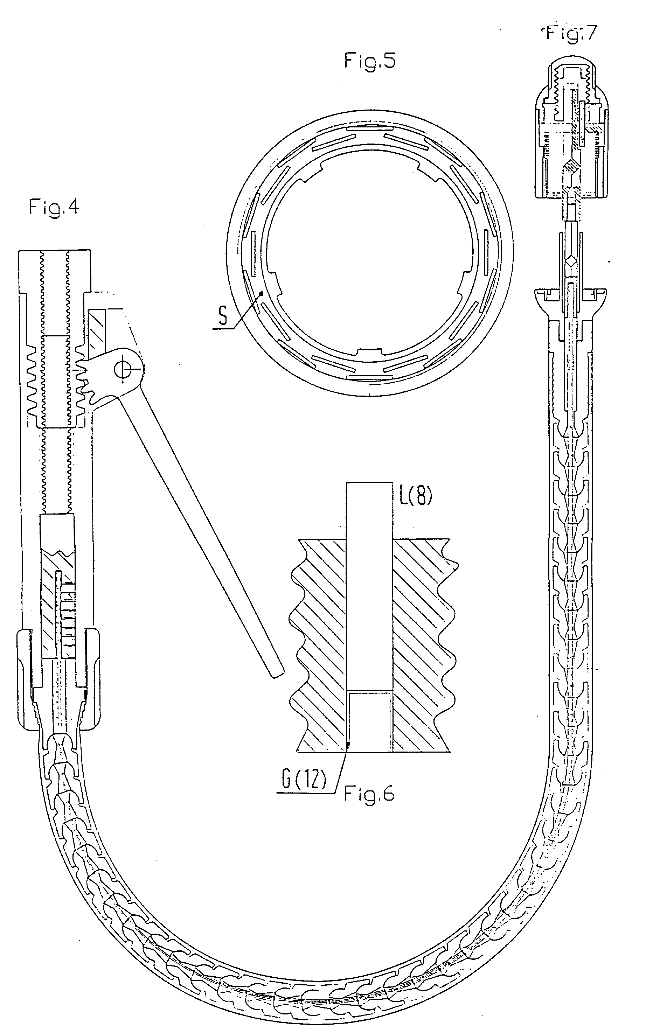

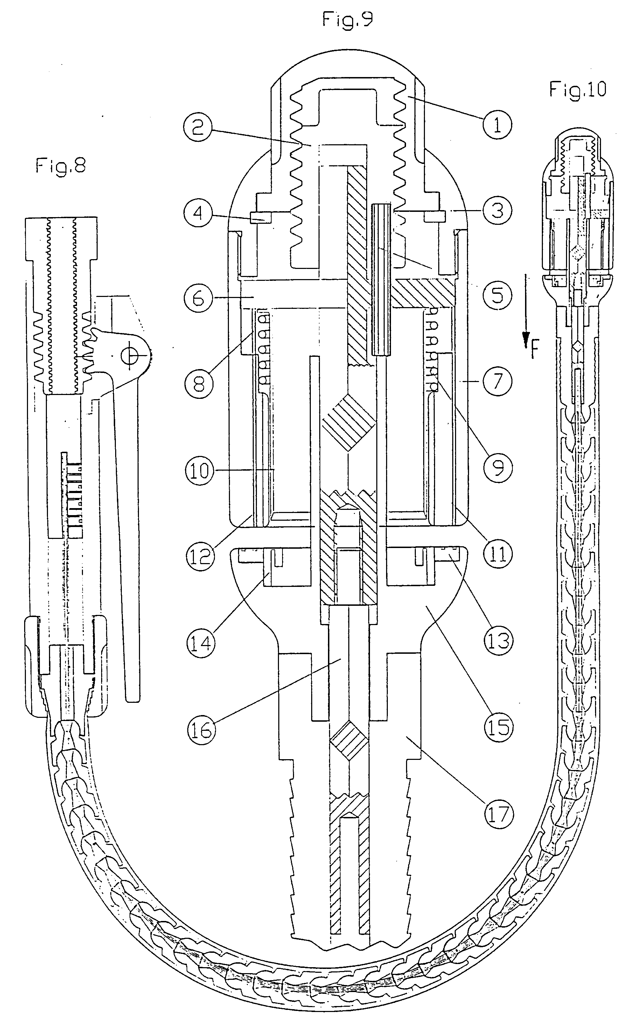

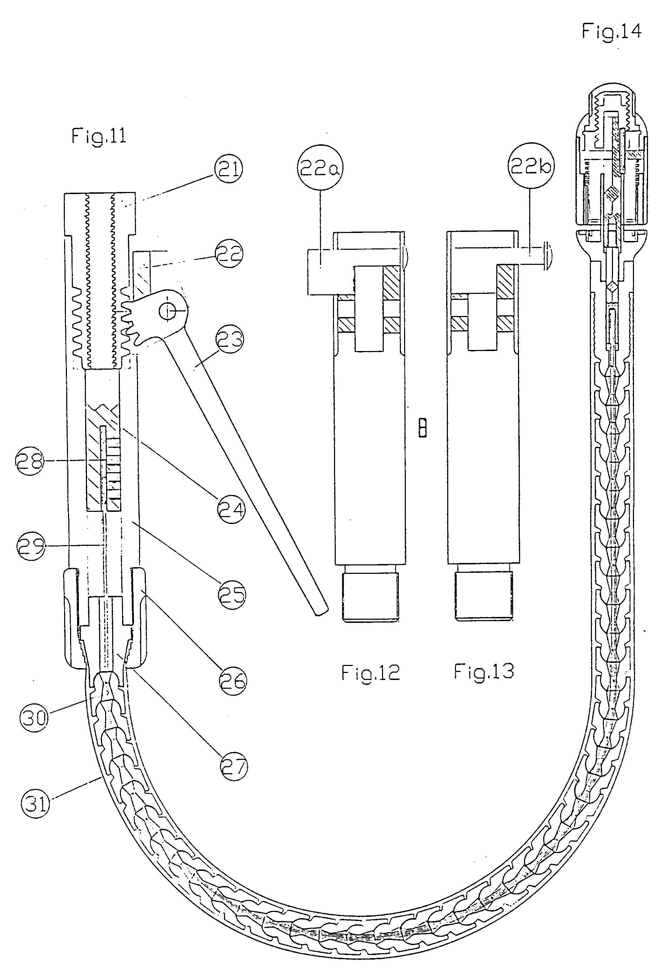

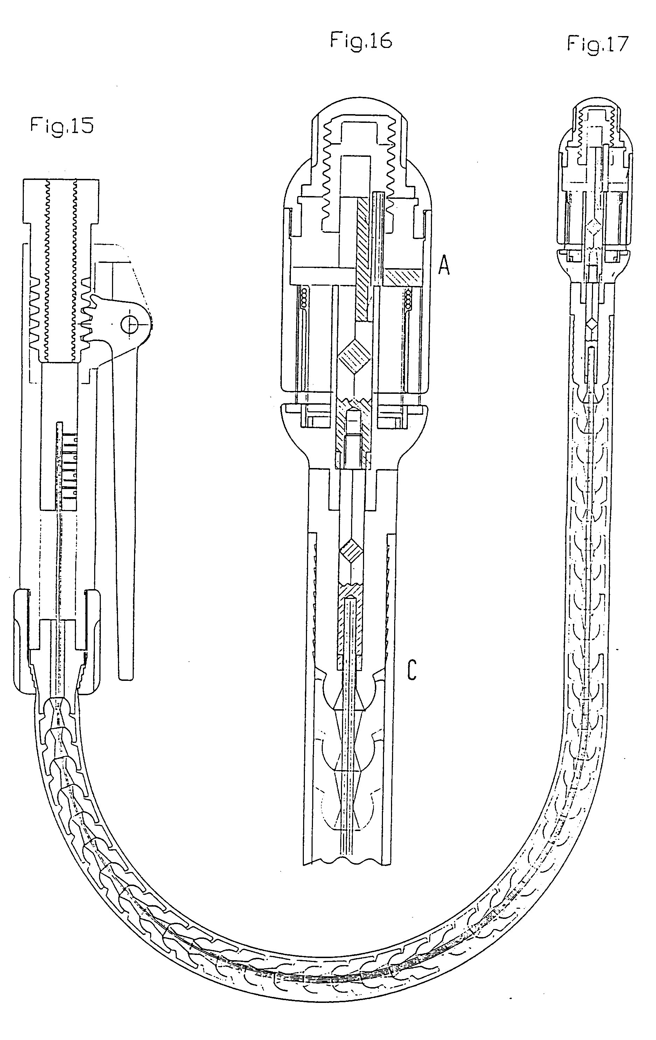

- Figures 1, 4, 8, 11 and 15 show the control device B (at the left end in the Figures) of the stapler according to the invention in following positions: the Figure 1 in rest position; the Figure 4 when the cartridge is opened for arranging the two sections of organic tissue to be sutured; the Figure 8, when stapling and punching are just made; the Figure 11, when the cartridge is preadjusted at the outside, e.g. by the surgeon or by his help using the pawl according to the thickness of the tissue to be sutured; the Figure 15 during the re-opening phase.
- Figures 3, 7, 9, 10, 14 and 16 represent the views in partial section of the operative part and anvil (part A at the other end), that is the stapler head in the corresponding positions specified for the control device; in particular: the Figure 3, the end of the stapler with suturing cartridge mounted (Figure 9); the Figure 7 the cartridge in position of use; the Figure 9, the components of the preadjusted cartridge, in enlarged scale; the Figure 10 the cartridge after stapling and punching (Figure 8), the Figure 4 the same preadjusted cartridge and the Figure 16 the cartridge after the punching with yielding of the safety ring 14 (Figure 9);
- Figure 2 shows the details of the stapler end without head but with cap 32 that is used in the introduction phase through an organic opening, (e.g. rectum);
- Figure 5 is a view from the bottom of layout of the seats S of the staples G (details 11 and 12 of Figure 9);
- Figure 6 is a lateral section view of the cartridge with view of the staple G in its seat S and the tongue L, (detail 8 of Figure 9) that pushes the staple G;
- Figures 12 and 13 represent the safety system 22 (Figure 11) when inserted, respectively disengaged, and the Figures 18 and 19 are two block schemes representing the conventional method, respectively according to the present invention.

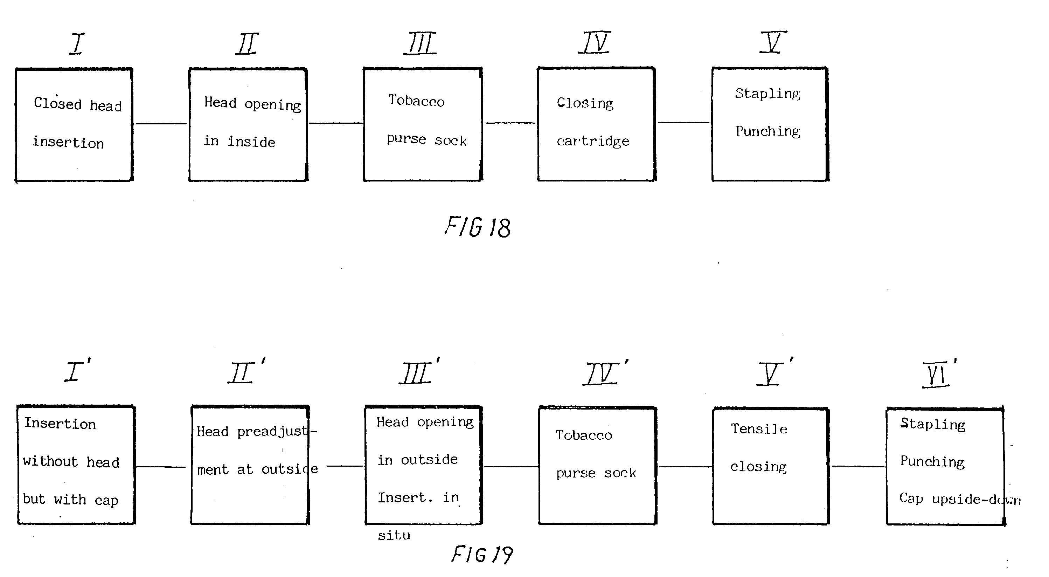

The apparatus includes at the left end a device B) controlling the stapler 21-22-23, an intermediate drive means c 31 and a means A) for application of the staples at the other right end (in drawings). Starting from this last means represented really in the right side of the Figures 3, 7, 10, 14, 17 in a reduced scale, but at the centre of the drawing sheets of the Figures 9 and 16 and with enlarged scale, it includes a graduated pushbutton or pawl 1 for the preadjustment of the thickness of the organic tissue to be stapled; a threaded cursor 2 for establishing the minimum closing distance of the cartridge, whose main body is indicated by 7 and whose external sheath is indicated by 3; a ring 4 preferably made of stainless steel for holding the pawl 1; the pushing cylinder 5 between the base 15 and the said cursor 2; and 6 the mobile plane for the output of the tongues 8 (that on their turn control the staplers G) and the hollow punch 10.

As above said, the cartridge includes the main body 7, the spring 9 for holding in position the hollow punch 10 and the cursor 2, and the tongues 8 for controlling the external 11 and internal 12 staples (G).

Under the lower base of the said head it is located the anvil or ring 13 for riveting the staplings 11-12 the safety ring 14 and the connection base 15 of the cartridge.

The transmission means (C) between the control device B) (that shall be described in the following) and the anvil head A) is represented in better details and with enlarged scale in the Figures 2 and 16. It includes the external sheath 31, the closing cap 32, a tie rod 18 with shaped end 16, the body 17 for the screwing of the cartridge and the hinged junctions 19.

The Figure 2 shows the interface between the transmission means C) 30-31 and the part A), that is the head + anvil 7-13.

The Figures 1 and 11 represent in detail the control device B) including a toothed lever 23 engaged, as a rack, with the external tooth of a pawl 21 for row adjustment (with long step) for the opening of the cartridge 7. (The Figure 3 shows the end of the stapler with staple cartridge A mounted and the Figure 7 represent the cartridge in use position). Turning to the Figure 1, the reference 24 shows the lower end of the tie rod 29 (extension of 18), while 28 shows the mechanical device for coupling the said tie rods 29 and 24.

The lower part of insertion of the flexible cable (transmission means) into the control device B) includes the ring nut 26 for locking the sheath 31 and a corresponding end of hinge connection 27. The control device B includes also a safety element 22 that, in the Figure 12, assumes the position 22a when it is inserted while the Figure 13 shows the position 22b when it is released.

The Figure 5 shows from the bottom the layout of the seats S of the staples G (corresponding to the details 11 and 12 of Figure 9) while the Figure 6 is a lateral section of the cartridge with view of the staple G into its seat S and the tongue (8 of Figure 9) that pushes the said staple.

Considering what exposed in the introduction regarding the general running of staplers, the apparatus running according to the invention shall be described shortly as follows:
According to the thickness of the intestine of the patient to be operated, the operator adjusts the distance between the anvil (13) and the main body (7) acting onto the graduated pushbutton (1).

Advantageously the adjustment is made out of the operation field (even if it is made in situ). Through the hole or opening available in the human body (e.g. the rectum opening for the intestine) the operating part of the apparatus is introduced (as represented in Figure 9) without cartridge with protection cap 3 mounted.

After having prepared the part to be stapled, e.g. the intestine, the said protection cap is removed (3) and the head is screwed.

Using the knob (21) located on the lever handle (23) the main body (7) is driven away. e.g. until 25 mm distance for making easier the insertion into the intestine and the related bag closing.

Moving the said knob located on the lever handle the main body 7 is approached to the anvil 13 into the direction of the arrow F.

The intestine cannot be squashed more that the space left free during the preadjusting phase. When it arrives at the stop, the knob shows a high resistance. By operating the lever it is moved the cursor (6) pressing the spring 9. The tongues 8 urge the staples G towards the anvil 13 closing them. Contemporaneously the hollow punch (10) cuts the intestine and breaks the safety ring 14 signaling that the operation is over.

A first feature is related to the fact that now the head can be removed and initially, that is, at the insertion, it is removed and adjusted from the outside.

According to a second feature the head is driven pulling in the direction of the arrow F. Initially the knob (23) is turned in clockwise direction, and the knob acting onto the wire 18 pulls it, therefore the body 7 is moved to the left side until the pins o similar ones of limit stop 5 are laid onto the lower body 15, that is onto the related thrust bearings. At this moment the operator feels a resistance to the further progress of the head in the direction F, this resistance being caused by the spring 9 that is introduced onto the hollow punch 10.

As above mentioned, at this moment the rotation of the knob 23 becomes hard, it is released 23 and operated the rack lever 21 that continues with multiplied force, the traverse of the hollow punchs 10; the same lever pushes contemporaneously the staples 12 until they are brought against the anvil 13; in this manner it starts the bending of the staples 12, that have just crossed the two edges of the tissue and continuing the stroke the punch end 10 cuts the said intestine tissue 14, alerting the surgical operator that the operation is over and that the staples 12 are now closed around the edges of the tissue. This cutting position is signaled acoustically by the sound "tac" showing the yielding of the ring and mechanically by the limit stop.

Furthermore, it is used a flexible pipe including a series of spherical hinges 30 slightly pressed and embedded (preferably made of polyammide plastics).

The ring nut 26 locks the sheath 31 while 27 is the end of the spherical hinge. It is essential that the play between the wire 28 and the zone of lesser diameter is reduced to a minimum value.

The flexible pipe can be covered by a sheath 31. For operating the lever 21 at the end of the rotation of the knob 23 the lever must be unlocked operating a wedge 22, that releases the lever.

The positions of the safety device are indicated by 22a and 22b.

Now the block scheme shown in fig. 19 in I′ shows that the apparatus is inserted without head and with protecting cap, therefore the diameter is practically the diameter of the flexible pipe and can be also inferior than 15 mm. In II′ it is made the preadjustment of the head at the outside, while in III′, IV′, V′, VI′ the classic phases of opening of the head, tobacco purse sock, cartridge closing and stapling and hollow punch are repeated.

For resuming, the main characteristics are the detachable head and the preadjustment thereof at the outside, and also the characteristics arising from the fact that: the head works in the contrary direction (with respect to above mentioned US patent), that is by drawing and not by compression, the control is one only, that is it includes a sole wire; the flexible pipe is a body that can be bent until 90° and does not tend to rectify under any minumm stress - the apparatus runs with a cartridge upside down, that is the fixed part is used as anvil and the mobile part is used as hammer - the cartridge is mounted by the surgeon at the last moment in the stapling zone after the flexible part has made the path along the duct and is gone out between the sections in the zone to be stapled, this action allowing to insert, into the intestine or in other pipe or duct, a device having an inferior diameter (about an half diameter) that damages in a limited manner the hole and the same pipe. It exists a safety system that signals to the surgeon, by a change of effort, that the punching of the intestine is occurred.

The apparatus is also advantageous as it can be sterilized completely mounted and in a sole phase by ethylene oxide (instead of using the differentiated sterilizations for the single parts by steam and/or solutions according to the well-known technics).

## Claims

1. An apparatus for applying surgical staples in surgical operations, including: A) at one end a head part for the application of said staples, comprising at least a cartridge (7) with associated staples and hollow punches (10) and an anvil (13); B) at the other end a control part provided with knob (21) and lever (23); and C) a flexible intermediate part for transmitting controls and stresses from B) to A), characterized in that said head part is detachable from the end (17) of the flexible intermediate part which is provided with a removable protective cap (32) having a diameter smaller than the diameter of the head and practically equal to the diameter of said flexible part which is thus taken to the operation zone through the duct to be stappled without said head, where the head is to be reattached for stapling the duct.

2. Apparatus according to claim 1, characterized in that the anvil (13) is also detachable from the cartridge (7), is engageable with the flexible intermediate part end freed from the removable cap, and has a distance from the cartridge adjustable according to the thickness of the duct wall.

3. Apparatus according to claim 1, caracterized in that the cartridge head works by drawing, its lower fixed part (15) acting as an anvil (13) and its upper downwards drawn mobile part (6) acting as a hammer.

4. Apparatus according to claim 3, characterized in that said head fixed part acting as an anvil is provided with a safety ring (14) which is broken by the hollow punch (10) at the closure of the staples (G).

5. Apparatus according to claim 1, characterized in that the part C) is formed by a tie rod (18) and by a series of hinges (30) formed by braked articulated joints made of plastic material, that guarantee a flexibility until 90° and keep the configuration assigned thereto until a stress for changig the position thereof is applied.

6. Apparatus according to claim 5, characterized in that the hinges are made of polyammide plastics and are covered by sheaths.

## Patentansprüche

1. Vorrichtung zum Vernähen von Anastomosen bei chirurgischen Operationen, mit: A) an einem Ende einem Kopfteil zur Ausführung der Vernähungen, der wenigstens ein Klammermagazin (7) mit entsprechenden Klammern und hohlen Lochinstrument (10) und einen Amboss (13) einschliesst; B) am anderen Ende, einem Führungsteil mit Griff (21) und Hebel (23); und C) einem biegsamen zwischenliegenden Antriebsteil für den Antrieb der Steuerungen und Beanspruchungen von B) bis A),
dadurch gekennzeichnet, dass gennnter Kopfteil vom Ende (17) des zwischenliegenden biegsamen Teil abnehmbar ist, welcher biegsame Teil mit Schutzkappe (32) mit einem kleinerem Durchmesser als der Kopfdurchmesser und praktisch gleich dem Durchmesser des gannanten biegsamen Teils ausgerüstet ist, der also in die zu operierende Zone durch den zu nähenden Kanal ohne den gennanten Kopf geführt wird, wobei der Kopf in genannter Zone wieder verbunden wird, um den Kanal zu nähen.

2. Vorrichtung gemäss Anspruch 1, dadurch gekennzeichnet, dass auch der Amboss (13) von dem Klammermagazin (7) abnehmbar ist, mit dem von der Kappe befreiten Ende des biegsamen zwischenliegenden Teils engagierbar ist und einen Abstand von dem Klammermagazin aufweist der je nach Dicke der Kanalwand einstellbar ist.

3. Vorrichtung gemäss Anspruch 1, dadurch gekennzeichnet, dass der Kopf mit Klammermagazin mit Zugkraft arbeitet, wobei sein unterer fester Teil (15) als Amboss (13) wirkt, und sein oberer beweglicher nach unten (6) gezogen Teil (15) als Hammer wirkt.

4. Vorrichtung gemäss Anspruch 3, dadurch gekennzeichnet, dass der feste als Amboss wirkende Teil mit einem Sicherheitsring (14) ausgerüstet ist, der von dem Holhlinstrument (10) bei Schliessung der Klammer (G) gebrochen wird.

5. Vorrichtung gemäss Anspruch 1, dadurch gekennzeichnet, dass der Teil C) mit einer Zugstange (18) ausgerüstet ist und von einer Serie von aus Kunststoff bestehenden Gelenken (30) geformt ist, die eine Biegsamkeit bis 90° erlauben und die zugewiesene Gestaltung beibehalten, bis kein Änderungskraft ihnen eingeprägt wird.

6. Vorrichtung gemäss Anspruch 5, dadurch gekennzeichnet, dass die Gelenke aus Polyamid-Kunststoff hergestellt und von Mänteln bedeckt sind.

## Revendications

1. Appareil pour exécuter des sutures chirurgicales dans les opérations chirugicales, incluant: A) à une de ses extrémités, une partie de la tête pour réaliser les sutures comprenant au moins une cartouche (7) contenant les agrafes et les emporte-pièces creux (10) et une enclume de pliage (13); B) à l'autre extrémité, une partie de commande équipée d'une poignée (21) et d'un levier (23); et C) une partie intermédiaire flexible de transmission des commandes et des sollicitations de B) vers A), caractérisé en ce que ladite partie de tête est amovible de l'extrémité (17) de la partie intermédiaire flexible qui est munie d'un capuchon de protection (32) ayant un diamètre plus petit que celui de la tête et pratiquement égal à celui de ladite partie flexible qui est ainsi amenée à la zone de l'opération à travers le conduit à suturer, sans ladite tête, cette dernière étant connectée de nouveau dans ladite zone pour suturer le conduit.

2. Appareil selon la revendication 1, caractérisé en ce que l'enclume (13) est désinsérable de la cartouche (7), peut être engagée avec l'extrémité de la partie intermédiaire flexible, libérée par le capuchon, et a une distance de la cartouche réglable selon l'épaisseur de la paroi du conduit.

3. Appareil selon la revendication 1, caractérisé en ce que la tête avec cartouche travaille en traction, la partie fixe agissant comme une enclume (13) et sa partie supérieure (15) mobile vers le bas (6) en agissant comme un marteau.

4. Appareil selon la revendication 3, caractérisé en ce que la ladite partie fixe de tête agissant comme une enclume est munie d'un anneau de sécurité (14) qui est rompu de l'emporte-pièce creux (10) pendant la fermeture des agrafes (G).

5. Appareil selon la revendication 1, caractérisé en ce que la partie C) est formée par un tirant (18) et par une série de joints sphériques (30) formés par des joints articulés en matière plastique, qui lui donnent une flexibilité jusqu'à 90° et maintiennet la configuration attribuée jusqu'à ce que l'on ne leur impriment un effort de modification.

6. Appareil selon la revendication 5, caractérisé en ce que les joints d'articulation sont formés en matière plastique de polyamides et sont couverts par des gaines.
